# EUROPEAN PATENT APPLICATION

(11) **EP 4 316 569 A2**
(43) Date of publication of application: **07.02.2024**
(21) Application number: 23213822.2
(22) Date of filing: 24.01.2019
(51) Int. Cl.: A61N 1/05

(54) **SYSTEMS AND METHODS FOR THE TREATMENT OF ORAL MALADIES USING ELECTRIC CURRENT**

(30) Priority: 24.01.2018 US 201862621099 P
(62) Divisional of application: 19744011.8
(71) Applicant: Biolectrics LLC, Cleveland, OH 44114 (US)
(72) Inventor: Mandel, David, Washington, DC 20001 (US); Leimkuehler Jr., William J, Westlake, OH 44145 (US); Nemeh, Issam, Westlake, OH 44145 (US); Cornelius, Steven, Rocky River, OH 44116 (US); Corn, David, Gates Mills, OH 44040 (US)
(74) Representative: Potter Clarkson

(57) **Abstract**

An apparatus and method for the concurrent treatment of multiple oral diseases and defects while promoting general oral hygiene utilizing direct current electricity. Electrodes are used to deliver a direct current to the gingival tissues of a mouth in order to achieve a number of therapeutic, prophylactic, and regenerative benefits. These benefits include killing oral microbes, increasing oral vasodilation, reducing oral biofilm, improving oral blood circulation, reversing oral bone resorption, promoting oral osteogenesis, treating gum recession, and fostering gingival regeneration. Other benefits include the treatment of gingivitis, periodontitis, and oral malodor, and other systemic diseases correlated with oral pathogens.

## Description

### Background

This invention relates to a method of concurrently promoting general oral hygiene, treating periodontal diseases such as gingivitis and periodontitis, killing oral microbes including cavity-causing bacteria, reducing oral biofilms, increasing blood flow in oral tissues, increasing salivation, promoting gingival tissue regeneration, fostering osteogenesis in the boney structures of the teeth, mouth and related areas, treating systemic diseases associated with oral bacteria, and treating other periodontal and oral maladies through the non-invasive application of weak direct current electricity to the surfaces in the oral cavity, and it also relates to an apparatus suitable for providing direct current electricity for these therapeutic, prophylactic, and regenerative effects.

Periodontal disease has been identified as a risk factor for various systemic diseases by both dentists and physicians. Included in these diseases are cardiovascular disease, adverse pregnancy outcomes, and diabetes with newfound evidence supporting its association with pancreatic diseases and arthritis. While many of the studies establish correlation between the presence of periodontal disease and these systemic conditions, causation, with most of these conditions, is still a subject of ongoing research. A few of the biological mechanisms which have been proposed as to how oral bacteria stemming from periodontal disease can cause systemic disease are as followed:
*1. Direct effect of oral infections:* Oral microbes and their byproducts can gain systemic access via the circulatory system through traveling through compromised tissue and inflamed periodontium in the oral cavity. In gaining systemic access, oral microbes have the potential to directly influence subclinical mediators of various systemic diseases.
2. *Inflammation*: People with periodontal disease have elevated levels of systemic inflammatory markers due to the burden of increased levels of oral bacteria. Treatment for periodontal disease has been reported to decrease systemic inflammation levels.
3. *Cross*-*reactivity*: The progression of systemic diseases can be accelerated by the immune response to bacterial heat-shock proteins creating antibodies that cross-react with innate heat shock proteins expressed on cells of the damaged tissues.

### Cardiovascular Disease

Studies investigating the potential association between periodontal disease and cardiovascular diseases, including atherosclerosis, coronary heart disease, and stroke have found a significant positive correlation between poor oral health and the prevalence of cardiovascular disease. While both diseases share several common risk factors, recent studies suggest that periodontitis may precede and therefore contribute to atherosclerotic complications. In fact, meta-analyses show that subjects suffering from periodontitis experience an increased risk for developing cardiovascular diseases.

While it has not been definitively shown if these bacteria initiate atherosclerosis or rather invade an already compromised artery, antibodies to periodontal bacteria, including *Fuseobacterium nucleatum* and *Streptococcus oralis,* have been found in blood serum and are associated with an increased risk of coronary heart disease. A mouse study found that intravenous inoculation with *Porphyromonas gingivalis* accelerated atherosclerotic development. Further, following oral inoculation, *P. gingivalis* DNA was found in the aortic tissue of those infected mice that showed observable signs of accelerated early atherosclerosis. Another study has named *F. nucleatum* as a synergistic agent with *P. gingivalis. F. nucleatum* enhances the ability of *P*. *gingivalis* to invade host cells due to a coaggregating effect between the two organisms. This is significant as bacteria within the atheroma may lead to the development of atherosclerotic plaque. The evidence thus far supports the idea that periodontitis leads to systemic exposure to oral bacteria which serves as a potential source of systemic inflammatory mediators, cytokines produced in the infected periodontal tissues, capable of initiating or worsening atherosclerosis and coronary heart disease when they enter into the blood stream. Clinical studies on periodontal disease have also revealed a positive association with coronary disease and emphasis is now being placed on understanding the exact relation between periodontal disease and atherosclerosis.

### Pre-term Birth

*Fusobaceterium nucleatum,* one of the most prevalent species of bacteria found in amniotic fluid and placental infections that cause preterm birth, is also often named the sole infectious agent in preterm labor with intact fetal membranes. *F. nucleatum* is also highly associated with various types of periodontal disease. During periodontal infection, when the oral mucosa is injured and inflamed and the quantities of periodontal pathogens increase dramatically, transient levels of bacteria can appear in the blood leading to selective colonization of undesired sites. One study demonstrated that pregnant mice injected hematogenously with *F. nucleatum* isolated from either amniotic fluid infection or an oral source resulted in fetal death.

Recently, a human stillbirth case was analyzed and it was found that the *F. nucleatum* did indeed originate from the mother's oral cavity, a fact that had not yet been proven. It is likely that the *F. nucleatum* translocated from the mother's mouth via the blood stream where it was then able to cross the endothelium to proliferate and colonize within the fetal membranes, amniotic fluid and fetus whereupon its presence lead to fetal demise. In a mouse model, hematogenous injection of *F. nucleatum* into pregnant mice resulted in specific bacterial colonization in the placenta causing localized inflammation. *F. nucleatum* was completely cleared from the maternal circulation after 24 hours of injection. However, once colonized in the immune privileged placenta, the bacteria proliferated quickly and caused fetal death within 3 days. Chronic periodontal disease could mediate infection through the translocation of periodontal bacteria/inflammatory markers to the fetoplacental unit.

### Diabetes

Diabetes mellitus is an endocrine disease that stems from genetic, environmental and behavioral risk factors. For the past several decades, diabetes has been considered a modifying factor for periodontal disease with recent years suggesting a bidirectional relationship between the two. Further, presence of periodontal disease has been implicated as a risk for diabetic complications, namely poor glycemic control. Recent longitudinal and systemic studies have seen periodontal disease correlated to higher risks of death from ischemic heart disease, diabetic nephropathy, end-stage renal disease and increased insulin resistance compared to patients with mild or no periodontal disease. In type II diabetes, insulin resistance is linked to the actions of pro-inflammatory cytokines. It is believed that periodontal disease leads to a significantly higher amount of these serum markers of inflammation, thus conferring insulin resistance. A human study examining the bacterial content of adults with and without type II diabetes found diabetic patients had significantly more severe periodontitis and higher levels of many oral bacteria, including *Streptococcus oralis.*

### Pyogenic Liver Abscess

*F. nucleatum* has recently been implicated in pyogenic liver abscess (PLA). Normally caused by biliary tract pathology, diverticular disease and bowel malignancy, atrophic gastritis and cryptogenic liver disease, PLA caused by *F. nucleatum* is very rare with *Escherichia coli, Klebsiella* and *Enterobacter* being the most commonly isolated microorganisms in the drained abscesses. *F. nucleatum* was found in the liver abscess with no other infectious source being found, except for a dental extraction. It is hypothesized that due to the coaggregation properties of *F. nucleatum,* it is able to transport and breach the mucosa of the colon and lead to bacteremia which results in hepatic abscess.

### Osteomyelitis

Osteomyelitis is a bone infection caused by bacteria, fungi or other germs. Commonly, bacteria spreads to the bone from infected skin, muscles or tendons and often time occur under a skin sore. The infection can also start in another part of the body and spread hematogenously. Occasionally *Fusobacterium* species have been isolated from bone/joint infections in the head and neck area and were associated with chronic periodontitis. A recent study has reported a case of osteomyelitis caused by *F. nucleatum* in conjunction with muscle abscess. The patient had no known predisposing factors and had no other infection sources except a history of periodontal disease. It is believed that due to the patient's poor oral hygiene, *F. nucleatum* bacteremia may have developed and lead to a hematogenous osteomyelitis of the lower leg.

### Arthritis

Numerous clinical studies have suggested a potential association between rheumatoid arthritis (RA) and periodontal disease as several oral bacteria species, such as *P. gingivalis* and *Prevotella intermedia,* have been isolated from the synovial fluid of patients. Periodontal disease is thought to allow bacteria to penetrate through the permeable pocket epithelial in the oral cavity to reach the underlying gingival connection tissue. From there, it may be transported out into the bloodstream with the ability to colonize elsewhere within the body. The oral bacteria found in the synovial fluid of patients suffering from RA has been attributed to synovial inflammation favorably trapping oral bacteria DNA, which suggests periodontal disease may have a perpetuating effect on joint diseases. Therefore, periodontitis may in fact be a factor leading to the autoimmune inflammatory responses characteristic of RA. Patients suffering from RA may also be at a higher risk of developing periodontal disease thus suggesting a bidirectional relationship between the two conditions. One particular study examined the presence of bacterial DNA in the synovial fluids of native and failed prosthetic joints of patients suffering from arthritis. Out of the 5 patients where bacterial DNA was found, *F. nucleatum* was detected in 4 of these 5 patients. This suggests that this bacterium can translocate from the oral cavity to the synovial fluid, as *F. nucleatum* was also found in the patient's plaque sample.

### Oral Biofilm

Periodontitis, gingivitis, and caries are infectious diseases of the oral cavity in which oral biofilm and bacteria plays a causative role. Biofilm formation is also involved in the pathogenesis of dental implant failures such as peri-implantitis, denture stomatitis, and oral yeast infections such as candidiasis. Oral biofilms begin with dental pellicle formation on the teeth. This pellicle is composed of salivary proteins that coat the exposed surfaces of the teeth, primarily the supra-gingival ones, to which the planktonic bacteria begin to adhere. The aerobic bacteria, including gram-positive cocci, such as *S. oralis,* are the early colonizers that begin forming the initial biofilm colony, primarily through cellular division of the adherent bacteria.

Once the initial colony has been established, other co-aggregating bacteria species, such as *F. nucleatum, P. gingivalis,* and other gram-negative, anaerobic bacteria attach to the previously formed colonies. As these colonies mature, they grow to cover the sub-gingival surfaces of the teeth and begin to induce inflammation in the periodontium.

US 2015/044628 discloses apparatus for cosmetic treatment of teeth and in particular, to an apparatus for whitening teeth. The apparatus comprises dental trays in which a chemical whitening composition is located. Energy-applying elements in the trays emit energy into the whitening composition to accelerate the teeth whitening activity of the teeth whitening composition.

### Summary of the Invention

Methods and apparatus are described for aiding overall oral health, and more particularly to treating periodontal diseases such as gingivitis, periodontitis, and peri-implantitis; killing oral microbes including cavity-causing bacteria; reducing oral biofilms; increasing blood flow in oral tissues; increasing salivation; promoting gingival tissue regeneration; fostering osteogenesis in the boney structures of the teeth, mouth, and related areas; treating systemic diseases associated with oral bacteria; and treating other periodontal and oral maladies through the non-invasive application of weak direct current electricity to the surfaces in the oral cavity.

In accordance with the present invention, there is provided a device comprising:
a mouthpiece sized and configured to fit into a mouth of a human, the mouthpiece including a first U-shaped channel;
a plurality of electrodes being electrically coupled to a controller and being supported by the mouthpiece, each electrode comprising one of an anodic electrode or a cathodic electrode, at least one electrode being disposed on a first surface of the channel and a second electrode being disposed on a second surface of the channel, the first surface and the second surface being separated by a width of a top surface or a bottom surface of a bite plane, wherein:
   the mouthpiece comprises an encapsulant encasing a flex circuit comprising electrically conductive silicone; and
   each electrode being encapsulated by the electrically conductive silicone and electrically coupled to a connector through the flex circuit.

### Brief Description of the Drawings

Figure 1 is a perspective view of a treatment apparatus according to the present invention.
Figure 2 is a top perspective view of a mouthpiece according to the present invention.
Figure 3 is a bottom perspective view of a mouthpiece according to the present invention.
Figure 4 is a front perspective view of a flat pattern according to the present invention.
Figure 5 is a bottom perspective view of a flat pattern according to the present invention
Figure 6 is a top plan view of a flex circuit according to the present invention.
Figure 7 is a bottom plan view of a flex circuit according to the present invention.
Figure 8 is a top perspective view of a bite plane according to the present invention.
Figure 9 is a bottom perspective view of a bite plane according to the present invention.
Figure 10 is a top plan view of a cable according to the present invention.
Figure 11 is a front elevation view of a controller according to the present invention.

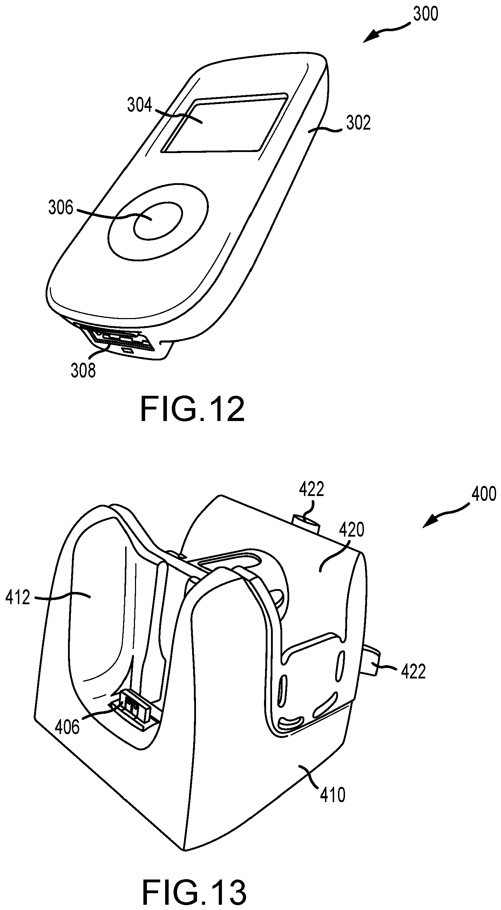

Figure 12 is a bottom perspective view of the controller shown in Figure 11.
Figure 13 is a perspective view of a charging station according to the present invention.

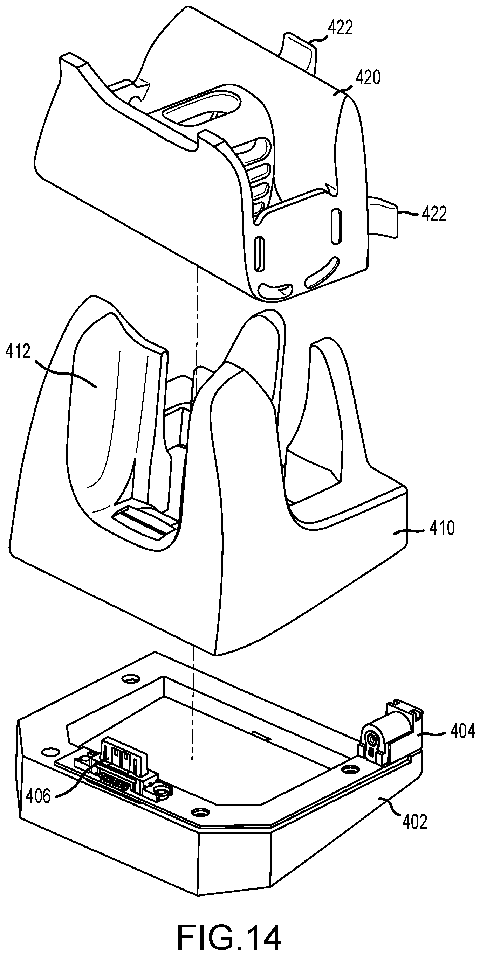

Figure 14 is an exploded perspective view of the charging station shown in Figure 13.

### Detailed Description

Although the disclosure hereof is detailed and exact to enable those skilled in the art to practice the invention, the physical embodiments herein disclosed merely exemplify the invention which may be embodied in other specific structures. While the preferred embodiment has been described, the details may be changed without departing from the invention.

It is known in the art that oral bacteria cannot survive when exposed to low-microampere direct current electricity. This method of killing oral bacteria and treating bacteria-caused conditions such as gingivitis has been demonstrated in Nachman, U.S. Pat. No. 4,244,373 of Jan. 13, 1981 and in Detsch, U.S. Patent 4,509,519 of Apr. 9, 1985. US Patent Application No. 2009/0117513 also discloses apparatus for the treatment of multiple oral diseases and defects utilizing direct current electricity. Killing oral bacteria has the added benefit of preventing tooth decay and dental caries, or cavities. Generally, tooth decay is attributed to aerobic acid-producing bacteria whose acid causes uncompensated demineralization of the teeth. However, Nachman does not instruct optimal approaches to reducing oral bacteria including aerobic and anaerobic bacteria on a species-by-species level and instead teaches a generic, untargeted treatment.

While researching the effect of direct current electricity on the mouth, the applicants discovered that by increasing the current level to the approximate range of 50 to 250 microamperes (µA), a direct current electrical treatment was able to deliver new and unexpected therapeutic, prophylactic, and regenerative benefits previously unknown in the art.

Specifically, by utilizing a direct current in the aforementioned range, not only did such a treatment kill bacteria, but it was also found to kill or disable viruses and fungus as well. Studies from the podiatric field have shown that higher current levels than those used in existing oral electrical treatments are necessary to effectively treat fungal infections ("Low-Voltage Direct Current as a Fungicidal Agent for Treating Onychomycosis", Kalinowski, et al., Journal of the American Podiatric Medical Association Vol. 94 No.6: 565-572,2004). By applying this knowledge of increased current levels from research outside the art, the applicants were able to add fungicidal and viricidal benefits to a method already known to be bactericidal. The applicants' studies have shown that these microbicidal properties begin to take effect within approximately 5 and 15 minutes of treatment, reducing both supra- and sub-gingival microbes.

In addition, the applicants' clinical research unexpectedly demonstrated that a direct current in the approximate range of 50 to 250 microamperes was able to regenerate gingival tissues, providing a non-surgical treatment alternative for those with recessed gums. While the osteogenic properties of electricity have been known in the art, the connection between nonosseous tissue regeneration and electricity were not well known in the art prior to these experiments. The unique current range associated with the method and apparatus of this invention is one of a few effective methods in the dental field to accomplish effective gingival tissue regeneration in a non-surgical manner.

In further research, the applicants conducted preclinical testing that examined the effects of direct current stimulation on three different oral bacteria (*F. nucleatum, S. oralis, P. gingivalis*) in both saline and saliva solutions. This testing varied the current levels, inoculum size of bacteria, solution medium, and treatment time to develop an optimal treatment to reduce these three bacteria species associated with both periodontal and systemic diseases.

The results of this testing yielded unexpected results and showed that each different bacterium had a different dose response to DC stimulation. Through this testing, the applicants identified treatment parameters that were able to kill up to 100% of *S*. *oralis,* 99.10 of *F*. *nucleatum,* and 52.3% of *P. gingivalis* in a single treatment lasting thirty minutes or less. This research yielded specifications for DC-based treatments of targeted pathogens that was previously unknown in the art. The optimal treatment parameters discovered in this research and described in this method can provide an innovative way to reduce these three species of bacteria, in both supra- and sub-gingival environments, and thus prevent and/or treat their associated complications including periodontal disease, biofilm formation, as well as the systemic diseases correlated to these oral pathogens.

In addition, scanning electron microscopy (SEM) was conducted on *F. nucleatum* colonies before and after a 30 minute treatment, according to the method of this invention, to better understand the mechanism by which the method according to this invention is able to reduce bacterial levels. The SEM imagery suggested that the method according to this invention interferes with bacterial cellular division and can weaken the outer envelope (cell membrane) resulting in fragile cellular structures that can easily break. It is contemplated that this phenomenon is an example of electroporation, where the permeability of cellular membranes may be affected by electrical stimulation either temporarily or permanently. It is further contemplated that the electroporation caused by the method according to this invention could play a role in developing new therapies in molecular biology which would take advantage of this cellular permeability and introduce new material into the cells of oral pathogens or oral tissues through mechanisms including, but not limited to, genetic material (transfection) such as DNA, RNA, sRNA, siRNA, plasmids, etc. These effects would prove a new tool in targeted gene therapies for oral applications.

Specifically, the method according to the present invention has been shown to reduce viable colony forming units (CFU) in various oral bacteria.

Table 1 below shows the efficacy of treatment according to the present invention at current levels of 50 µA or 500 µA for 5-, 10-, 20-, and 30-minute durations for bacterial cultures ranging from 10⁴ to 10⁷ colony forming units (CFU) of *Streptococcus oralis* in a saline solution.

**Table 1**

| In Vitro Efficacy of Device Against *Streptococcus oralis* in Saline | | | | | | |
|---|---|---|---|---|---|---|
| CFU | µA | 0 Min | 5 Min | 10 Min | 20 Min | 30 Min |
| | 50µA | 1120 | 1080 | 600 | 320 | 280 |
| 10e4 | 500µA | 1120 | 1200 | 800 | 240 | 0 |
| | 50µA | 10000 | 9600 | 8400 | 9200 | 7600 |
| 10e5 | 500µA | 11600 | 10400 | 11200 | 10800 | 8400 |
| | 50µA | 80000 | 63200 | 52800 | 32400 | 24800 |
| 10e6 | 500µA | 80800 | 70000 | 15200 | 14000 | 15600 |
| | 50µA | 1280000 | 1080000 | 1040000 | 800000 | 440000 |
| 10e7 | 500µA | 1080000 | 520000 | 160000 | 120000 | 320000 |

Table 2 below shows the efficacy of treatment to the present invention at current levels of 50 µA or 500 µA for 5-, 10-, 20-, and 30-minute durations for bacterial cultures ranging from 10⁴ to 10⁷ CFU of *Streptococcus oralis* in a saliva solution.

**Table 2**

| In Vitro Efficacy of Device Against *Streptococcus oralis* in Saliva | | | | | | |
|---|---|---|---|---|---|---|
| CFU | µA | 0 Min | 5 Min | 10 Min | 20 Min | 30 Min |
| | 50µA | 160 | 160 | 80 | 80 | 40 |
| 10e4 | 500µA | 200 | 80 | 80 | 80 | 80 |
| | 50µA | 5600 | 5600 | 6800 | 5600 | 4000 |
| 10e5 | 500µA | 8400 | 6800 | 7200 | 6400 | 2800 |
| | 50µA | 25600 | 25200 | 15200 | 17200 | 18400 |
| 10e6 | 500µA | 23600 | 16800 | 15600 | 17600 | 15200 |
| | 50µA | 316000 | 284000 | 300000 | 276000 | 220000 |
| 10e7 | 500µA | 324000 | 328000 | 300000 | 292000 | 252000 |

Table 3 below shows the efficacy of treatment to the present invention at current levels of 50 µA or 500 µA for 5-, 10-, 20-, and 30-minute durations for bacterial cultures ranging for 10⁴ and 10⁶ CFU of *Fusobacterium nucleatum* in a saline solution.

**Table 3**

| In Vitro Efficacy of Device Against Fusobacterium nucleatum in Saline | | | | | | |
|---|---|---|---|---|---|---|
| CFU | µA | 0 Min | 5 Min | 10 Min | 20 Min | 30 Min |
| | 50µA | 480 | 280 | 280 | 120 | 40 |
| 10e4 | 500µA | 560 | 440 | 400 | 200 | 120 |
| | 50µA | 94000 | 91600 | 85600 | 70400 | 84400 |
| 10e6 | 500µA | 46400 | 45600 | 27200 | 2000 | 400 |

Table 4 below shows the efficacy of treatment according to the present invention at current levels of 50 µA or 500 µA for 5-, 10-, 20-, and 30-minute durations for bacterial cultures ranging from 10⁴ to 10⁶ CFU of *Fusobacterium nucleatum* in saliva.

**Table 4**

| In Vitro Efficacy of Device Against *Fusobacterium nucleatum* in Saliva | | | | | | |
|---|---|---|---|---|---|---|
| CFU | µA | 0 Min | 5 Min | 10 Min | 20 Min | 30 Min |
| | 50µA | 1480 | 1480 | 1560 | 680 | 880 |
| 10e4 | 500µA | 2360 | 2360 | 1720 | 1240 | 1080 |
| | 50µA | 19600 | 19600 | 15200 | 14400 | 14000 |
| 10e5 | 500µA | 18000 | 17200 | 14400 | 11200 | 10800 |
| | 50µA | 348000 | 112000 | 120000 | 72000 | 68000 |
| 10e6 | 500µA | 156000 | 128000 | 124000 | 32000 | 28000 |

Table 5 below shows the efficacy of treatment to the present invention at current levels of 50 µA or 500 µA for 5-, 10-, 20-, and 30-minute durations for bacterial cultures ranging for 10⁵ CFU of *Porphyromonas gingivalis* in a saline solution.

**Table 5**

| In Vitro Efficacy of Device Against *Porphyromonas gingivalis* in Saline | | | | | | |
|---|---|---|---|---|---|---|
| CFU | µA | 0 Min | 5 Min | 10 Min | 20 Min | 30 Min |
| | 50µA | 3440 | 2040 | 2720 | 1640 | 1640 |
| 10e4 | 500µA | 2440 | 2120 | 2200 | 1880 | 1840 |

Thus, this method and corresponding apparatus are able to achieve multiple prophylactic, therapeutic, and regenerative effects whose combination was not previously known or available in the art. Namely, these effects are: promotion of oral osteogenesis, destruction or disabling of oral microbes, gingival tissue regeneration, reduction and prevention of the formation of oral biofilms, caries prevention, increased oral vasodilation and oral blood flow, treatment of common oral conditions such as gingivitis and periodontitis, treatment of systemic diseases and conditions correlated with oral pathogens, and generally improved oral hygiene.

These effects are accomplished by the delivery of direct current to the gingiva through a plurality of electrodes in direct contact with gingival tissue surfaces (e.g., lingual, buccal, palatal, and/or vestibular gingival tissue). The electrodes may be fashioned out of any electrically-conductive material, including but not limited to metals such as silver, stainless steel, copper, gold, platinum, palladium, aluminum, an alloy thereof, electrically-conductive nanotubes, carbonized rubber, electrically-conductive silicone, or electrically-conductive polymers. The electrodes may be composed of the same or of differing materials. These electrodes fit snuggly against the lingual and buccal sides of the gingiva and make direct contact with each side of the gingiva to pass direct current electricity across the teeth and neighboring gingival tissues.

The electrodes on each side (lingual or buccal) of the gingiva are of the same polarity. Electrodes on opposite sides of the gingiva are of the opposite polarity. This allows the current to flow across the teeth and gums to the electrodes positioned on the transverse gingiva to complete the electrical circuit. Put another way, all electrodes on the lingual side of the gingiva will be completely anodic or completely cathodic. All electrodes on the buccal surfaces of the gingiva, transverse the lingual surfaces of the gingiva, would have the opposite polarity. The polarization of these electrodes may be reversed during treatment or in between treatments.

The mandibular and maxillary gingiva each have a set of a plurality of polarized electrodes as previously described. This allows for treatment of both the maxillary and mandibular periodontium either simultaneously or in isolation. The maxillary and mandibular sets of electrodes may be powered by two different adjustable power supplies or by the same adjustable power supply.

Electrical conductors then connect these electrodes to an adjustable power supply. All of the anodic electrodes will connect to the positive pole of the power supply and all of the cathodic electrodes will connect to the negative pole of the power supply. The adjustable power supply is capable of delivering a stable, direct current in the approximate range of 1 to 500 microamperes. The preferred current setting for most treatments is in the approximate range of 50 to 250 microamperes.

In order to increase conductivity in the tissues adjacent to the electrodes, an ionic or colloidal liquid or gel may be used as a conductive medium to decrease electrical resistance in the mouth. This medium would be placed along any desired areas of desired electrical contact, such as the teeth, gums, or surrounding oral tissues. Examples of such a medium would include, but not be limited to, colloidal silver gel, liquid colloidal silver, colloidal copper gel, liquid colloidal copper, colloidal gold gel, liquid colloidal gold, saline gel, liquid saline or any combination thereof.

Colloidal silver, in whole or in combination, has great promise not only in increasing electrical current flow, but also in offering additional bactericidal benefits. Colloidal silver, in concentrations as little as five parts per million, is known to be bactericidal by inhibiting a bacterium's production of adenosine triphosphate.

This conductive medium may also contain dietary supplements including, but not limited to, oil of oregano. Oil of oregano is believed to have many health benefits and may also be microbicidal. Such microbicidal properties would be effective in treating common oral infections and diseases as well as aiding in preventative oral care.

This conductive medium may also contain teeth whitening agents. This would allow for the addition of teeth whitening to the list of benefits offered by an embodiment of this invention. A whitening agent that is catalyzed by direct current electricity could be included and may even offer reduced teeth whitening treatment times when compared with nonelectrically-catalyzed whitening agents.

Artificial or natural flavorings may also be added to this conductive medium to offer a more appealing taste to the user, similar to the method of flavoring dental fluoride treatments. This flavoring would mask any unpleasant tastes from the ingredients of the conductive medium or as well as any taste of the mouthpiece or electrodes themselves.

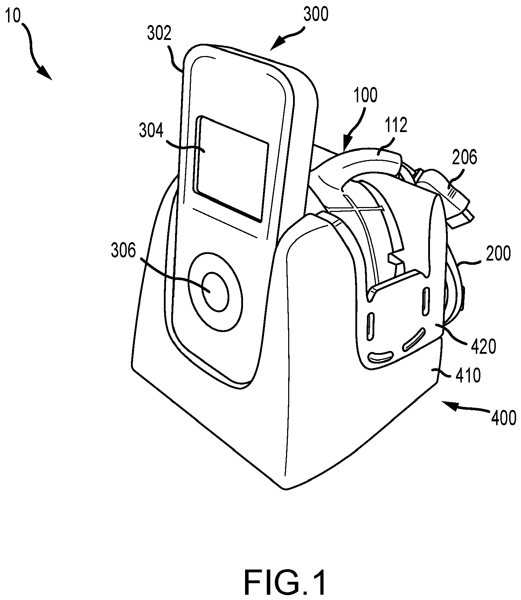

Figure 1 shows one embodiment of a treatment apparatus 10 according to this invention. The treatment apparatus 10 is preferably a stand-alone device comprising a mouthpiece 100, a controller 300, and a charging station 400.

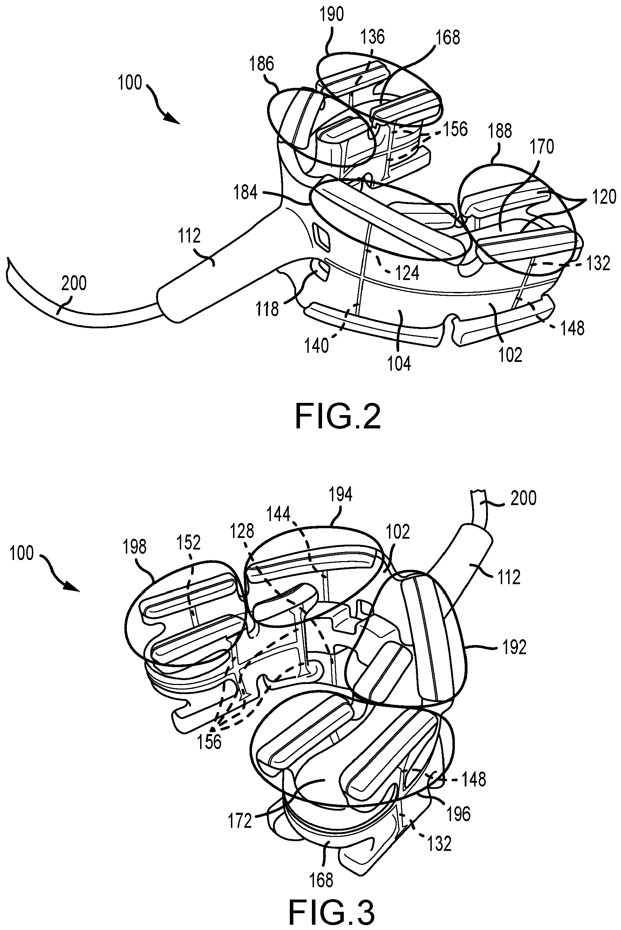

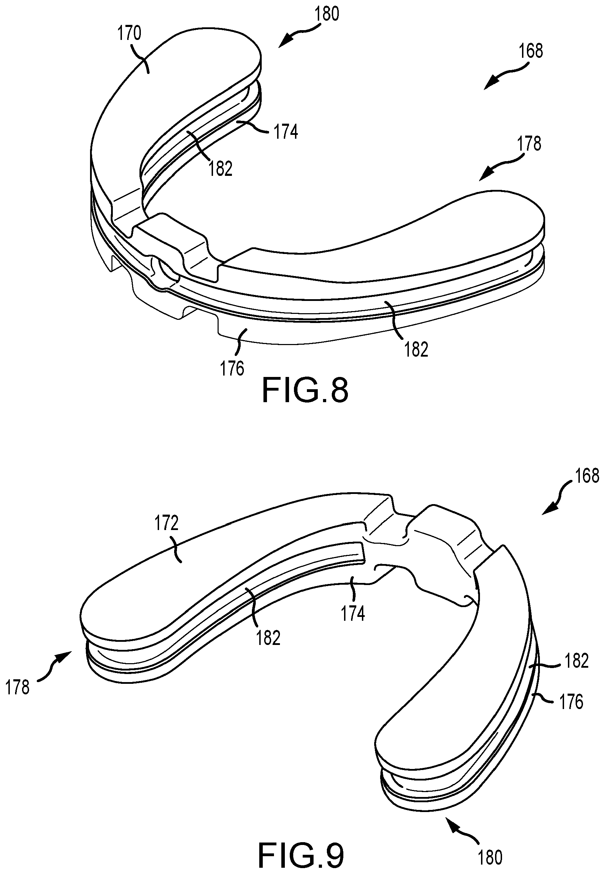

Looking at Figures 2 and 3, the mouthpiece 100 according to the present invention is shown. The mouthpiece 100 preferably comprises a flat pattern 102 and a bite plane 168 (see also Figures 8 and 9).

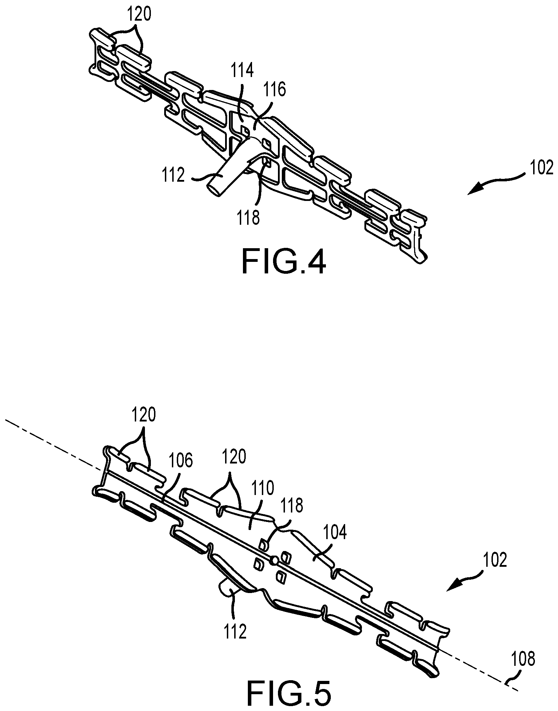

Figures 4 and 5 better show the flat pattern 102. The flat pattern 102 preferably comprises an encapsulant 104 encasing a flex circuit 122. Preferably the encapsulant 104 is a flexible polymer comprising a mixture of Silbione 4040AUI, Bluesil 4040 Activator and blue pigment. The flat pattern 102 preferably comprises a ridge 106 extending along a centerline 108 on an inside surface 110, a strain relief portion 112 extending outward from a center portion 116 of an outside surface 114, and a plurality of cutouts 118 extending from the inside surface 110 through the outside surface 114 approximate to the strain relief portion 112 configured to allow air passage to and from the user during use of the treatment apparatus 10. Preferably, the strain relief portion 112 curves downward when worn and can act as an indicator with respect to the proper orientation of the mouthpiece 100 during use.

The flat pattern 102 further comprises a electrically conductive polymer 120, preferably an electrically conductive silicone, provided at predetermined locations on the flat pattern 102 as discussed further below.

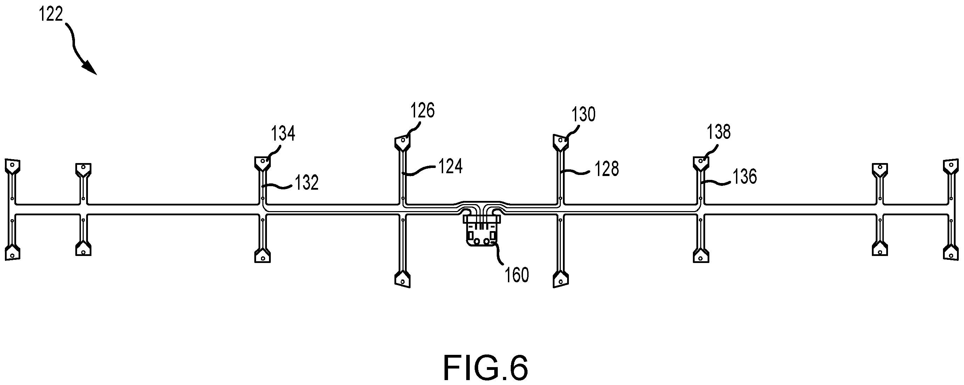

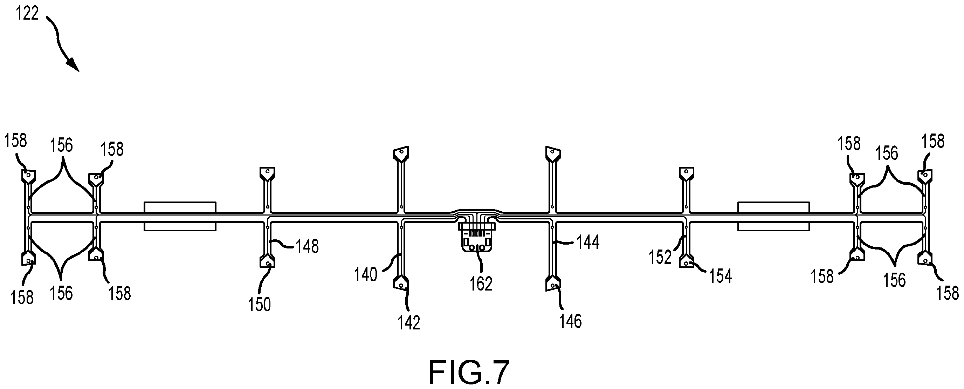

Figures 6 and 7 illustrate an exemplary embodiment of the flex circuit 122 according to the present invention. The flex circuit 122 is preferably formed from a copper-clad polyimide. The flex circuit 122 preferably comprises individual anodic electrodes 124, 128, 132, 136, 140, 144, 148, 152 (eight shown here) and a plurality of interconnected cathodic electrodes 156 (eight shown here) provided in a grid-like pattern.

It is preferable that the number of anodic electrodes is equal to the number of cathodic electrodes, but alternative arrangements are contemplated with different numbers of anodic and cathodic electrodes. Each anodic electrode 124, 128, 132, 136, 140, 144, 148, 152 has a distal end 126, 130, 134, 138, 142, 146, 150, 154, respectively, and each of the plurality of cathodic electrodes 156 has a distal end 158. Although shown as individual, controllable anodes and common cathodes, it is to be understood that targeted stimulation may be selectively provided, such as may be desirable to treat predetermined gingival areas. To provide targeted stimulation, delivery of electrical current to other portions of the mouth is preferably prevented or reduced mechanically or electrically. As an example, mechanical prevention or reduction may be achieved by particularized arrangement of electrodes, such as providing an anodic or cathodic electrode on a mouthpiece at a first location of gingival tissue that at least partially surrounds (a) a tooth to be removed and replaced with an implant, or (b) an empty tooth socket from which a tooth has already been removed intentionally or by accident, or (c) a portion of a previously placed dental implant. The mechanical prevention or reduction may be further enhanced by providing a cathodic or anodic electrode on the mouthpiece at a second location of (preferably on the opposite side of teeth from first location) gingival tissue that at least partially surrounds (a) the tooth to be removed and replaced with an implant, or (b) an empty tooth socket from which a tooth has already been removed intentionally or by accident, or (c) a portion of a previously placed dental implant. If the two electrodes are provided as described, and no other electrodes are disposed on the mouthpiece, then mechanical reduction of electrical current stimulation is achieved. In this way, a mouthpiece may be customized for a particular user by mechanically arranging electrodes on the mouthpiece to target electrical stimulation towards a dental implant site.

As an example of electrical prevention or reduction of non-targeted electrical current is selective electrode control by the controller. That is, mechanically there may be provided on a mouthpiece a plurality of electrodes spaced about the mouthpiece, as shown and described herein. However, through electrical control of such electrodes, each electrode may have a selectable state to provide stimulation. The selectable electrode state may be anodic, cathodic, or off (e.g., tri-stated). Thus, where targeted electrical current is desired, a first electrode on the mouthpiece may be selected to be an anodic or cathodic electrode. The first electrode position on the mouthpiece may correspond to a first location of gingival tissue that at least partially surrounds (a) a tooth to be removed and replaced with an implant, or (b) an empty tooth socket from which a tooth has already been removed intentionally or by accident, or (c) a portion of a previously placed dental implant. The electrical prevention or reduction of non-targeted electrical stimulation may be further enhanced by a second electrode on the mouthpiece being selected to be a cathodic or anodic electrode (opposite the first electrode). The second electrode position on the mouthpiece may correspond to a second location of (opposite side of teeth from first location) gingival tissue that at least partially surrounds (a) the tooth to be removed and replaced with an implant, or (b) an empty tooth socket from which a tooth has already been removed intentionally or by accident, or (c) a portion of a previously placed dental implant. If the two electrodes are selected as described, and no other electrodes are activated on the mouthpiece (e.g., all other electrodes are turned off or sent into a high impedance, or tri-state, mode), then electrical reduction of electrical current stimulation is achieved. In this way, a mouthpiece may be mechanically standardized for multiple users, but electrically customized to target electrical stimulation towards a dental implant site.

While mechanical and electrical prevention or reduction of stray or non-targeted electrical current has been described with respect to targeting a single dental implant site, it is to be understood that such targeting may be accomplished at multiple implant sites simultaneously or in a time sequenced fashion (e.g., one target site is stimulated for a predetermined time and then a different target site is stimulated for a predetermined amount of time).

Figure 6 shows a top view of the flex circuit 122 wherein a first group of anodic electrodes (comprising anodic electrodes 124, 128, 132, 136) are each independently electrically connected to a first flat pattern connector 160. Figure 7 illustrates a bottom view of the flex circuit 122 wherein a second group of anodic electrodes (comprising anodes 140, 144, 148, 152) are independently electrically connected to a second flat pattern connector 162 along with the plurality of cathodic electrodes 156. It should be noted that use of a single connector, in place of the first and second flat pattern connectors 160, 162, is also contemplated and should be understood to be within the purview of the present invention. The configuration described allows for each of the anodic electrodes 124, 128, 132, 136, 140, 144, 148, 152 to be independently energized. Though, as described above, every electrode on the mouthpiece may be programmable, in which case the common cathodic connection 156 would be replaced with separate connections to the connector 162. It is to be understood that the only exposed conductive surfaces on the flex circuit 122 are the distal ends of the electrodes and the electrical contacts on the connectors 160,162. The interconnections between the distal ends and the connectors are encased in a copper clad laminate, bondply or coverlay.

As stated above, the flex circuit 122, along with the first and second flat pattern connectors 160, 162, are preferably substantially encased in the encapsulant 104. The distal ends 126, 130, 134, 136, 140, 144, 148, 152 of the anodic electrodes 124, 128, 132, 136, 140, 144, 148, 152, respectively, and the distal ends 158 of each of the plurality of cathodic electrodes 156 are preferably coated with the conductive polymer 120 (see Figure 5). Preferably, the conductive polymer 120 is exposed only on the inside surface 110 (i.e., the treatment side) of the flat pattern 102 and therefore is configured to not make unwanted contact with other tissues, like the lips, gums, and/or cheek of a patient.

The bite plane 168 is shown in greater detail in Figures 8 and 9. The bite plane 168 preferably comprises a flexible polymer, similar if not the same as the material comprising the flat pattern 102 (i.e., preferably a mixture of Silbione 4040AUI, Bluesil 4040 Activator and blue pigment), and is substantially u-shaped to follow the general teeth pattern of a human user. The bite plane 168 has a top surface 170, a bottom surface 172, an inside surface 174, an outside surface 176, a left portion 178, and a right portion 180. A groove 182 preferably extends along the outside surface 176 and a majority of the inside surface 174.

The groove 182 of the bite plane 168 is configured to receive the ridge 106 of the flat pattern 102 (see Figure 5) and is preferably secured with an adhesive (not visible). Whereby, when assembled, the plurality of cathodic electrodes 156 is positioned proximate to the inside surface 174 of the bite plane 168, with distal ends 158 of the individual cathodic electrodes extending above the top surface 170 and below the bottom surface 172. The anodic electrodes 124, 128, 132, 136, 140, 144, 148, 152 are positioned proximate to the outside surface 176 with the distal ends 126, 130, 134, 138 above the top surface 170 and the distal ends 142, 146, 150, 154 below the bottom surface 172 of the bite plane 168 opposite a respective cathodic electrode of the plurality of cathodic electrodes 156. Preferably, the conductive polymer 120 is in contact with the distal ends 126, 130, 134, 138, 142, 146, 150, 154 of each of the anodic electrodes 124, 128, 132, 136, 140, 144, 148, 152 and the distal ends 158 of each of the plurality of cathodic electrodes 156, individually and separately. The flat pattern 102 is preferably sized and configured for the conductive polymer 120 to be positioned at or near the gingival margin (not shown), in physical contact with gingival tissue (and preferably not in physical contact with teeth) in the user's mouth (i.e., where the gums meet the surface of the teeth), whereby the anodic electrodes 124, 128, 132, 136, 140, 144, 148, 152 span the exterior (buccal and/or vestibular) gingival surfaces of the mouth and the plurality of cathodic electrodes 156 span the interior (lingual and/or buccal) gingival surfaces of the mouth, in this configuration.

The arrangement of opposing anodic and cathodic electrodes defines eight treatment zones (here shown as treatment zones 184, 186, 188, 190, 192, 194, 196, 198) which may be independently controlled as discussed further below.

Looking at Figure 2 (and also Figure 3 for reference) it is shown that treatment zone 184 comprises anodic electrode 124 and an opposing cathodic electrode of the plurality of cathodes 156, treatment zone 186 comprises anodic electrode 128 and an opposing cathodic electrode of the plurality of cathodes 156, treatment zone 188 comprises anodic electrode 132 and an opposing cathodic electrode of the plurality of cathodes 156, and treatment zone 190 comprises anodic electrode 136 and an opposing cathodic electrode of the plurality of cathodes 156.

Looking at Figure 3 (and also Figure 2 for further reference) it is shown that treatment zone 192 comprises anodic electrode 140 and an opposing cathodic electrode of the plurality of cathodes 156, treatment zone 194 comprises anodic electrode 144 and an opposing cathodic electrode of the plurality of cathodes 156, treatment zone 196 comprises anodic electrode 148 and an opposing cathodic electrode of the plurality of cathodes 156, and treatment zone 198 comprises anodic electrode 152 and an opposing cathodic electrode of the plurality of cathodes 156.

It is also contemplated that the electrode polarization of the mouthpiece 100 could be reversed at any time, even during the administration of treatment.

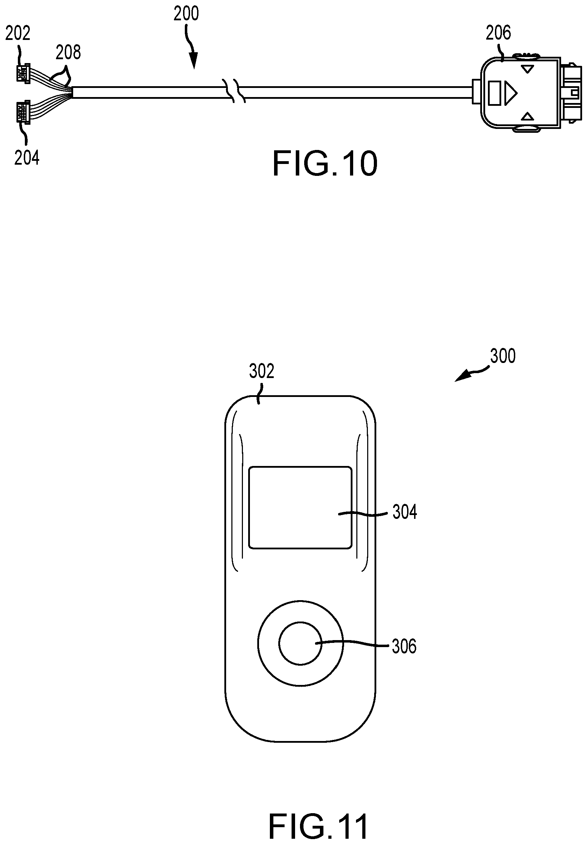

Figure 10 shows an exemplary embodiment of a cable 200 according to the present invention. The cable preferably comprises a first cable connector 202 and a second cable connector 204, both electrically connected to a third cable connector 206 through a plurality of conductors 208 configured to be associated with each of the anodic electrodes 124, 128, 132, 136, 140, 144, 148, 152 and the plurality of cathodic electrodes 156. The first cable connector 202 is configured to interface with the first flat pattern connector 160 and the second cable connector 204 is configured to interface with the second flat pattern connector 162. One or several electrical conductors provided in the cable 200 may be jacketed with silicone rubber, and the cable 200, itself, is preferably a silicone jacketed cable.

The controller 300 preferably comprises a body 302, a liquid crystal display (LCD) screen 304, a push button 306, a controller connector 308, and a printed circuit board (PCB) (hidden). The controller 300 preferably delivers direct current of a predetermined amplitude and/or at a predetermined frequency. Pulsed bi-phasic current, alternating current, or other current may also be used.

The operation of the controller 300 by a user (not shown) is preferably performed through the pressing of the push button 306. For example, the user can start or pause the delivery of current to the mouthpiece 100 by pressing the push button 306. To prevent unintentional operation, the duration of the pressing of the push button 306 is sensed.

Depending on the state of the controller 300, a press of the push button 306 can have multiple functions. For example, in an exemplary embodiment, when in an off state, a press-and-hold of the push button 306 for approximately 1.5 seconds will turn on the controller 300 and further enter into a ready state. When in the ready state, a press-and-release of the push button 306 will enter the controller 300 into a run state and start the output of current to the mouthpiece 100. Whereas, a press-and-hold of the push button 306 for approximately 3.0 seconds when in the ready state will shut the controller 100 off. When in the run state, a press-and-release of the push button 306 will enter the controller 300 into a pause state and pause the output of current to the mouthpiece 100. A press-and-hold of the push button 306 for approximately 3.0 seconds when in the run state will shut off the controller 300. When in the pause state, a press-and-release of the push button will return the controller 300 to the run state, and a press-and-hold for 3.0 seconds when in the pause state will shut off the controller 300. After the treatment program has completed, the controller 300 will enter a complete state and a press-and-hold of the push button 306 will shut off the controller 300.

The LCD screen 304 preferably indicates the status of the treatment apparatus 10 to the user. For example, the LCD screen 304 may display indications such as, "Ready to Treat," "Running," "Check," and "Fault." "Ready to Treat" indicates that the controller 300 is in the ready state and ready to begin the delivery of direct current to the mouthpiece 100. "Running" means that the controller 300 is in the run state and delivering electrical current to the mouthpiece 100.

Preferably, when in the run state, a timer countdown depicting remaining treatment time may be output to the LCD screen 304. The LCD screen 304 may also preferably display the electrical current set point, indicating the amount of electrical current being delivered to the mouthpiece 100 and/or amount of current being sensed by the controller 300. Preferably, this status is displayed for approximately 5.0 seconds out of every 30 seconds, but may be displayed during the entire run state. "Check" may indicate that an open circuit has been detected. "Fault" may indicate an over-current fault and/or a low battery voltage condition.

Preferably, a light emitting diode (LED) (hidden) will illuminate when the controller 300 is in the run state. A tone generator (e.g. buzzer, speaker, etc.) (hidden) preferably delivers an audible tone to indicate a change in state and/or status and may provide feedback to the user for button presses and configuration events (discussed further below).

The controller 300 is preferably configured by a clinician or other trained staff member prior to a user interfacing with the treatment apparatus 10. Additionally, or alternatively, the patient may configure the controller 300. Preferably configuration of the controller 300 is performed through attachment of an additional piece of hardware (not shown) connected to the controller 300, but may also take place through a wireless connection (e.g. Bluetooth^{®}, Wi-Fi, near field communications (NFC), infrared, magnetic). Finally, the controller 300 may be provided with a default treatment regimen to reduce or eliminate initial configuration effort by a clinician or patient.

Configuration parameters preferably include: selection of any combination of the treatment zones 184, 186, 188, 190, 192, 194, 196, 198 to provide direct current for treatment; selection of direct current output values, for example, 6µA, 12µA, 18µA, 25µA, 50µA, 62µA, 75uA, 100µA, 125µA, 150µA, and 200µA (preferably not to exceed 1,000pA total current across all treatment zones at any one time); and selection of treatment duration (preferably from 1 minute through 30 minutes selectable in increments of 1 minute).

The controller 300 is preferably capable of monitoring compliance of treatments performed by the treatment apparatus 10 and recording a number of performance metrics on an electrical erasable programmable read-only memory (EEPROM). The controller connector 308 is preferably configured to be compatible with the JTAG (Joint Test Action Group) standard to aid in accessing the EEPROM and the compliance records by a computer or other electronic device (not shown). The records may be utilized by a clinician (not shown) to evaluate and discuss the treatment.

For example, some metrics and data collected may include the following (along with the dates and times of such occurrences): the number of treatments started; the number of successfully completed treatments; the number of open circuit faults; the number of treatments with an open circuit; the number of treatments with an open circuit that still completed treatment successfully; the number of overcurrent faults; the number of low battery faults; the number of times the device was paused; the number of treatments that were paused but still completed treatment successfully; the number of times the user turns the device on; the number of times the device is powered off by the user; the number of times the device is powered off by software; and the total number of minutes the device has run since a memory reset.

The controller 300 may also be configured to dynamically monitor the electrical characteristics (i.e., resistance, voltage, current) of each treatment zone and adjust the treatment without clinician or user intervention. For example, if one of the anodic electrodes 124, 128, 132, 136, 140, 144, 148, 152 makes contact with one of the plurality of cathodic electrodes 156 through a metal filling or crown in the mouth of a patient and therefore completely bypasses the gingiva to be treated, the controller 300 may be configured to detect the artificially low resistance in the return current and disable the affected treatment zone.

A real-time clock (hidden) is also preferably included to record the time and date at which the metrics and data are collected.

The EEPROM will preferably store the following information about a currently running treatment: how many minutes of treatment (up to 30 minutes) the user completed of treatment; whether the mouthpiece was disconnected while running; whether an overcurrent fault occurred; and whether a low battery fault occurred; how many pauses the user initiated during treatment; and how many open circuit checks occur during the treatment.

It is also contemplated that the controller 300 may be configured to detect when the mouthpiece 100 is disconnected from the controller 300. This may be accomplished through a test of continuity between two pins on the third cable connector 206 of the cable 200 and the controller connector 308.

The controller 300 may also be configured to detect when, although there is a connection between the mouthpiece 100 and the controller 300, the mouthpiece 100 is not located in the mouth of a user. To do so, the controller 300 monitors the delivery of current and whether current is detected on any of the plurality of cathodic electrodes 156 (i.e., the return path). If no current is detected on the return path, the controller 300 pauses treatment and indicates an error on the LCD screen 304. For instance, the controller 300 monitors the stimulation circuits, including the anodic and cathodic electrodes. The controller 300 includes circuitry to measure or predict the amount of current to be delivered to the mouthpiece 100 (delivered current), and also to measure the amount of return current received from the mouthpiece 100 (return current). The circuitry then compares the return current to the delivery current, and if the difference is greater than a predetermined value (e.g., a percentage of the delivered current, such as 10% to about 50%), then stimulation is paused, preferably on all electrodes, and a fault message is displayed on the controller. Once the difference between delivered current and return current is less than the predetermined amount, then the stimulation program or regimen will resume from where it left off, preferably so no or little treatment time is lost.

Graph 1 below provides parameters for monitoring the current delivered to a treatment zone. As described above, the running current setpoint and also the duration "T" is determined and set during configuration prior to a patient using the treatment apparatus 10, with the recommended setting for duration "T" at two seconds. The open circuit is preferably approximately 80% of the running current set point and the over current fault limit is preferably 120% of the running current set point. The hardware limit is preferably approximately 200 to 300 µA per stimulation channel (e.g., per anodic electrode).

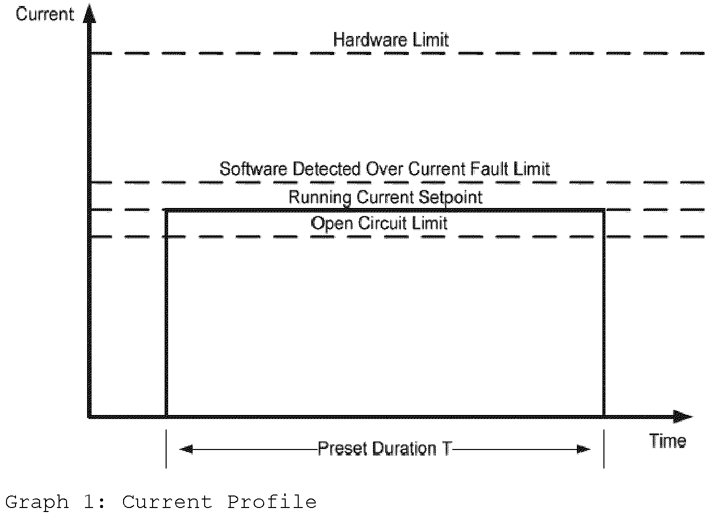

The current on the return path is preferably polled eight times per second when the controller 300 is delivering current to any of the treatment zones.

If the current detected on the return path is less than the open circuit limit in any of the treatment zones for more than the preset duration, all of the problematic treatment zones will pause and a notification will be displayed on the LCD screen 304 to check the mouthpiece 100. Additionally, or alternatively, when the current detected on the return path is less than the open circuit limit in any of the treatment zones for more than the preset duration, all treatment zones will pause and a notification will be displayed.

If the current detected on the return path is more than the over current fault limit in any of the treatment zones for more than the preset duration, current will be stopped to all treatment zones and the LCD screen 304 will display a fault notification.

It is further contemplated that the treatment apparatus 10 be fully compatible with wireless technology such as Bluetooth^{®} technology, near-field communication, and wi-fi to communicate with a user's electronic device (not shown), such as a cell phone, tablet, or personal computer. Preferably, a user may review usage history, the prescribed treatment plan, and/or a comparison of usage history versus treatment plan. The treatment apparatus 10 may also provide notifications regarding scheduled treatment sessions to any of the user's electronic devices. This functionality is contemplated as operating through an application (not shown) downloadable to a user's electronic device. The application may also be configured to share this data with a central server for storage, remote monitoring by the prescribing clinician, provide one-way or two-way communication between patient and clinician, and/or allow for a clinician to remotely adjust the treatment parameters. Additionally, firmware upgrades may be supplied to the controller 300 wirelessly.

Charging station 400 preferably comprises a base 402, a cradle 410, and a mouthpiece cup 420. The base 402 preferably comprises a power input 404 and base connector 406. The power input 404 is configured to receive input power from a power input source (not shown) (for example, a direct current transformer plugged into a standard electrical outlet providing alternating current). The base connector 406 is preferably configured to be received within the controller connector 308 and deliver power to a rechargeable power source (not shown) within the controller 300.

The cradle 410 is configured to be coupled with the base 402 and to removably receive the mouthpiece cup 420. The cradle 410 preferably has a pocket 412 sized and configured to removably receive the controller 300.

The mouthpiece cup 420 is preferably configured to hold the mouthpiece 100 and has a plurality of protrusions 422 around which the cable 200 may be wrapped.

In another embodiment of this invention or in combination with those previously described, an ionic or colloidal medium in the form of a liquid or a gel may be used to decrease electrical resistance in the mouth and to facilitate a more even current distribution across oral electrodes. Any combination of one or more ionic or colloidal compounds may be used. Examples of such a medium would include, but not be limited to, colloidal silver gel, liquid colloidal silver, colloidal copper gel, liquid colloidal copper, colloidal gold gel, liquid colloidal gold, saline gel, liquid saline or any combination thereof. Artificial or natural flavorings may be added to this medium to offer a more appealing taste to the user. The medium may also contain dietary supplements including, but not limited to, oil of oregano. This medium may also contain teeth-whitening chemical agents. A whitening agent that is catalyzed by the direct current would be most effective in this ionic or colloidal medium.

Thus, the reader will see that at least one embodiment addresses a desired need in the oral hygiene and dental fields to concurrently treat common oral diseases and conditions in a more effective, less invasive, and less expensive manner. These embodiments promote general oral hygiene, reduce oral biofilm, treat periodontal diseases such as gingivitis and periodontitis, kill oral microbes including bacteria and thus preventing cavities and tooth decay, increase vasodilation and blood flow in oral tissues, promote gingival tissue regeneration, foster osteogenesis in the boney structures of the teeth, mouth, and related areas, treat systemic diseases related to oral pathogens, and treat other periodontal and oral maladies through the non-invasive application of weak direct current electricity to the surfaces in the oral cavity.

In some cases, dental procedures can break up oral bacterial colonies found in biofilms and introduce bacteria into the bloodstream causing bacteremia and other infections. It is further contemplated that it may be desirable to utilize a mouthpiece according to the present invention immediately prior to performing a dental procedure. The treatment apparatus 10 according to the invention may be used by the patient either at home or in the dental office. In this manner, the living bacteria in the patient's mouth, both supra- and sub-gingival, can be reduced prior to the procedure and the risk of bacteremia and other infections will be reduced. For example, and not by way of limitation, the treatment apparatus 10 may be utilized prior to a dental prophylaxis or a scaling and root planning procedure in a dental office to reduce the risks of introducing bacteria into the patient's blood stream.

The treatment apparatus 10 may also be utilized following a clinical procedure as prevention for infections, for scenarios including but not limited to post-extraction or post-implantation infection prevention.

### Prevention of Systemic Disease

It is contemplated that a mouthpiece according to the present invention may be used to prevent or treat systemic diseases as will be outlined in more detail below. The method according to the present invention has been shown to be effective in reducing the amount of oral bacteria, specifically *F. nucleatum, P. gingivalis,* and *S. oralis.*

### 1. Cardiovascular disease

It is contemplated that use of a mouthpiece according to the present invention may be used to reduce microbial burdens caused by the translocation of oral bacteria, including but not limited to *S*. *oralis, P. gingivalis,* and *F. nucleatum,* from the gingival tissues to the rest of the body and also decrease the amount of inflammatory mediators produced by oral bacteria. Further, by reducing *F. nucleatum,* it is contemplated that the ability of *P. gingivalis* to invade host cells will be lessened and thus diminishing the development of bacteremia that has been linked with the initiation/worsening of atherosclerosis and coronary heart disease.

It is contemplated that a mouthpiece according to the present invention may be used according to a predetermined treatment regimen to prevent, treat and/or mitigate cardiovascular disease. In the predetermined treatment regimen, the patient will wear a mouthpiece according to the present invention for a predetermined amount of time at a predetermined current level and at predetermined time intervals. It is further contemplated that the specific treatment regimen may be determined based on the bacterial levels present in a patient. According to one embodiment of the invention, the treatment regimen would consist of a patient wearing a mouthpiece according to the present invention for 20 minutes once per day at a current level of 500µA. For acute cardiovascular conditions, this treatment may continue on a daily basis until the conditions is resolved. For chronic cardiovascular disease, this treatment may be repeated a few times a week on a continuing basis.

### 2. Still Birth

It is further contemplated that a treatment with a mouthpiece according to the present invention according to a predetermined treatment protocol would reduce the oral population of *F. nucleatum* associated with periodontal disease and thus prevent, treat and/or mitigate still birth. In turn, this reduction would lessen the likelihood of *F. nucleatum* translocating from the oral cavity into the bloodstream, where it could then migrate into the placenta and colonize. It is contemplated that a mouthpiece according to the present invention may be used according to a predetermined treatment regimen to prevent still birth. In the predetermined treatment regimen, the patient will wear a mouthpiece according to the present invention for a predetermined amount of time at a predetermined current level and at predetermined time intervals. It is further contemplated that the specific treatment regimen may be determined based on the bacterial levels present in a patient. According to one embodiment of the invention, the treatment regimen would consist of a patient wearing a mouthpiece according to the present invention for 20 minutes once per day at a current level of 500µA for the duration of the pregnancy. The treatment parameters outlined above have been demonstrated to be highly efficient at reducing levels of *S. oralis* and *F*. *nucleatum* at inoculation sizes of 10⁷ colony-forming units (CFU).

### 3. Diabetes

It is contemplated that a mouthpiece according to the present invention according to a predetermined treatment protocol may be used to prevent, treat and/or mitigate diabetes by causing a reduction of *S. oralis* in the oral cavity and consequently reduce the amount of serum markers of inflammation produced by bacterial infections. In the predetermined treatment regimen, the patient will wear a mouthpiece according to the present invention for a predetermined amount of time at a predetermined current level and at predetermined time intervals. It is further contemplated that the specific treatment regimen may be determined based on the bacterial levels present in a patient. According to one embodiment of the invention, the treatment regimen would consist of a patient wearing a mouthpiece according to the present invention for 20 minutes once per day at a current level of 500µA to effectively reduce oral levels of *S*. *oralis* that in turn will lower the amount of systemic inflammatory markers. This treatment may be repeated multiple times a week on an ongoing basis to help reduce inflammatory markers.

### 4. Pyogenic Liver Abscess

It is contemplated that a mouthpiece according to the present invention according to a predetermined treatment protocol may be used to prevent, treat and/or mitigate pyogenic liver abscess by causing a reduction of *F*. *nucleatum.* Specifically, it is contemplated that treatment with a mouthpiece according to the present invention would reduce bacterial levels and may stop *F*. *nucleatum* and other oral bacteria species from traveling to the liver and reduce overall bacteremia. In the predetermined treatment regimen, the patient will wear a mouthpiece according to the present invention for a predetermined amount of time at a predetermined current level and at predetermined time intervals. It is further contemplated that the specific treatment regimen may be determined based on the bacterial levels present in a patient. According to one embodiment of the invention, the treatment regimen would consist of a patient wearing a mouthpiece according to the present invention for 20 minutes once per day at a current level of 500µA to effectively reduce oral levels of F. *nucleatum* which may prevent any bacteria from being transported from the oral cavity systemically. This treatment may be repeated multiple times per week until the abscess is reduced.

### 5. Osteomyelitis

It is contemplated that a mouthpiece according to the present invention according to a predetermined treatment protocol may be used to prevent, treat and/or mitigate osteomyelitis by causing a reduction of *F. nucleatum.* In the predetermined treatment regimen, the patient will wear a mouthpiece according to the present invention for a predetermined amount of time at a predetermined current level and at predetermined time intervals. It is further contemplated that the specific treatment regimen may be determined based on the bacterial levels present in a patient. According to one embodiment of the invention, the treatment regimen would consist of a patient wearing a mouthpiece according to the present invention for 20 minutes per treatment at a current level of 500µA to effectively reduce oral levels of *F. nucleatum* bacteria and prevent any bacteria from being transported from the oral cavity systemically. This treatment may be used in conjunction with or separate from standard antibiotic-based treatments for osteomyelitis. When used in conjunction with antibiotics, treatment will normally continue for approximately 29 to 42 days. When used separately from antibiotics, this treatment may be used once a day for a few months for acute conditions, or a few times a week on a continuing basis for chronic conditions.

### 6. Arthritis

It is contemplated that a mouthpiece according to the present invention according to a predetermined treatment protocol may be used to prevent, treat and/or mitigate arthritis by causing a reduction of *F. nucleatum.* In the predetermined treatment regimen, the patient will wear a mouthpiece according to the present invention for a predetermined amount of time at a predetermined current level and at predetermined time intervals. It is further contemplated that the specific treatment regimen may be determined based on the bacterial levels present in a patient. According to one embodiment of the invention, the treatment regimen would consist of a patient wearing a mouthpiece according to the present invention for 20 minutes once per day at a current level of 500µA to effectively reduce oral levels of *F. nucleatum* bacteria and prevent any bacteria from being transported from the oral cavity and translocating to the synovial fluid and reducing the associated inflammation. This treatment may be repeated multiple times per week on a continual basis for this type of chronic condition.

### Reducing Biofilm and Preventing Biofilm Formation

It is contemplated that a mouthpiece according to the present invention according to a predetermined treatment protocol may be used to prevent, treat and/or mitigate oral biofilm by causing a reduction of *F*. *nucleatum, P. gingivalis, and*/*or S. oralis,* all of which are involved in oral biofilm formation. In the predetermined treatment regimen, the patient will wear a mouthpiece according to the present invention for a predetermined amount of time at a predetermined current level and at predetermined time intervals. It is further contemplated that the specific treatment regimen may be determined based on the bacterial levels of specific bacterial species present in a patient. According to one embodiment of the invention, the treatment regimen would consist of a patient wearing a mouthpiece according to the present invention for 20 minutes once per day at a current level of 500µA to effectively reduce oral levels of *F. nucleatum* bacteria to prevent further biofilm formation caused by *F. nucleatum* and to reduce the viability of existing biofilm colonies of *F. nucleatum.*

According to another embodiment of this invention, the treatment regimen would consist of a patient wearing a mouthpiece according to the present invention for 20 minutes once per day at a current level of 50µA to effectively reduce oral levels of *P*. *gingivalis* bacteria to prevent further biofilm formation caused by *P. gingivalis* and to reduce the viability of existing biofilm colonies of *P. gingivalis.*

Furthermore, according to another embodiment of this invention, the treatment regimen would consist of a patient wearing a mouthpiece according to the present invention for 20 minutes once per day at a current level of 500µA to effectively reduce oral levels of *S. oralis* bacteria to prevent further biofilm formation caused by *S. oralis* and to reduce the viability of existing biofilm colonies of *S. oralis.*

These treatments for biofilm reduction and prevention may be repeated on a daily basis for three to six weeks for acute biofilm-based issues or may be repeated once or more per week on a continuing basis for chronic biofilm issues.

### Treatment and/or Prevention of Peri-Implantitis

Peri-implantitis is generally inflammation of oral tissue in physical contact with, surrounding, or otherwise in proximity to, and occurring after, placement of a dental implant. This inflammation may be reduced or prevented using methods according to the present invention. Methods may be performed before and/or after a dental implant surgical procedure of dental implant placement or replacement.

A method of reducing a likelihood of peri-implantitis involves, prior to a dental implant being placed or replaced partially or in its entirety, applying or directing electrical current to gingiva tissue near or at an oral site of future implantation. While electrical current may be distributed elsewhere throughout oral tissue, at least 6 µA and more preferably at least approximately 50 µA of electrical current (and preferably no more than 300 µA) is delivered to the gingiva tissue near or at a predetermined oral site of future implantation. A pre-surgery treatment regimen may consist of approximately twenty minutes of electrical stimulation per day for one to fourteen days prior to a dental implant surgical procedure.

A method of reducing a likelihood of peri-implantitis (if it has not yet begun) or reducing peri-implantitis (if it has already begun) involves, after a dental implant has been placed or replaced partially or in its entirety, applying or directing electrical current to gingiva tissue near or at an oral site of implantation. While electrical current may be distributed elsewhere throughout oral tissue, at least 6 µA and more preferably at least approximately 50 µA of electrical current (and preferably no more than 300 µA) is delivered to the gingiva tissue near or at a predetermined oral site of implantation. A post-surgery treatment regimen may consist of approximately twenty minutes of electrical stimulation per day for one to fourteen days after a dental implant surgical procedure, or until desired inflammation reduction has occurred.

While the pre-surgery and post-surgery methods have been separately described for clarity, it is to be understood that either or (preferably) both methods may be utilized for a particular patient, or user of the mouthpiece.

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is described by the claims.

The following numbered statements are further disclosed:
1. A device (10) comprising:
   a mouthpiece (100) sized and configured to fit into a mouth of a human, the mouthpiece (100) including a first U-shaped channel; and
   a plurality of electrodes (124, 128, 132, 136, 140, 144, 148, 152, 156) being electrically coupled to a controller (300) and being supported by the mouthpiece (100), each electrode (124, 128, 132, 136, 140, 144, 148, 152, 156) comprising one of an anodic electrode and a cathodic electrode, at least one electrode (124, 128, 132, 136, 140, 144, 148, 152) being disposed on a first surface of the channel and a second electrode (156) being disposed on a second surface of the channel, the first surface and the second surface being separated by a width of a top surface (170) or a bottom surface (172) of a bite plane (168);
   wherein the mouthpiece (100) comprises an encapsulant (104) encasing a flex circuit (122); and
   each electrode (124, 128, 132, 136, 140, 144, 148, 152, 156) having a distal end (126, 130, 134, 138, 142, 146, 150, 154, 158) being encapsulated by the electrically conductive polymer (120) and electrically coupled to a connector (160,162) through the flex circuit (122) .
2. A device according to statement 1, further comprising:
   a strain relief portion (112) extending outward from a center portion (116) of an outside surface (114) of the encapsulant (104), and
   a plurality of cutouts (118) extending through the encapsulant (104) to allow air passage therethrough.
3. A device according to statement 1, the first U-shaped channel configured to receive one or more teeth of a human.
4. A device according to statement 3, the mouthpiece further comprising a second U-shaped channel, the second U-shaped channel configured to receive one or more additional teeth of the human, the second U-shaped channel being aligned with and opposing the first channel.
5. A device according to statement 4, wherein the first channel and second channel are at least partially defined by and separated by the bite plane (168) .
6. A device according to statement 4, wherein all electrodes (124,128,132,136,140,144,148,152) disposed on the first surface of each channel are selectively activatable to the same polarity.
7. A device according to statement 6, wherein all electrodes (124, 128, 132, 136, 140, 144, 148, 152) disposed on the first surface of both channels are selectively activatable to the same polarity.
8. A device according to statement 4, where all electrodes (156) disposed on the second surface of each channel are selectively activatable to the same polarity.
9. A device according to statement 8, wherein all electrodes (156) disposed on the second surface of both channels are selectively activatable to the same polarity.
10. A device according to statement 1, wherein the electrically conductive polymer (120) comprises an electrically conductive silicone.
11. A device according to statement 1, wherein each electrode (124,128,132,136,140,144, 148,152,156) is selectively programmable to a high-impedance mode.
12. A device according to any preceding statement, wherein the plurality of electrodes (124, 128, 132, 136, 140, 144, 148, 152, 156) comprises a plurality of pairs of electrodes,
   wherein each pair of electrodes is selectively activatable by the controller (300) separate and apart from any other electrode on the mouthpiece (100) .
13. A device according to statement 12, wherein the plurality of pairs is eight pairs.
14. A device according to statement 13, wherein four pairs are disposed along surfaces of each channel.
15. A device according to statement 1, wherein the first and second electrodes are selectively, simultaneously activatable in opposing polarities to establish a treatment zone (184, 186, 188, 190, 192, 194, 196, 198).

## Claims

1. A device (10) comprising:
a mouthpiece (100) sized and configured to fit into a mouth of a human, the mouthpiece (100) including a first U-shaped channel; and
a plurality of electrodes (124, 128, 132, 136, 140, 144, 148, 152, 156) being electrically coupled to a controller (300) and being supported by the mouthpiece (100), each electrode (124, 128, 132, 136, 140, 144, 148, 152, 156) comprising one of an anodic electrode and a cathodic electrode, at least one electrode (124, 128, 132, 136, 140, 144, 148, 152) being disposed on a first surface of the channel and a second electrode (156) being disposed on a second surface of the channel, the first surface and the second surface being separated by a width of a top surface (170) or a bottom surface (172) of a bite plane (168);
wherein the mouthpiece (100) comprises an encapsulant (104) encasing a flex circuit (122); and
each electrode (124, 128, 132, 136, 140, 144, 148, 152, 156) having a distal end (126, 130, 134, 138, 142, 146, 150, 154, 158) being encapsulated by the electrically conductive polymer (120) and electrically coupled to a connector (160,162) through the flex circuit (122) .

2. A device according to claim 1, further comprising:
a strain relief portion (112) extending outward from a center portion (116) of an outside surface (114) of the encapsulant (104), and
a plurality of cutouts (118) extending through the encapsulant (104) to allow air passage therethrough.

3. A device according to claim 1, the first U-shaped channel configured to receive one or more teeth of a human.

4. A device according to claim 3, the mouthpiece further comprising a second U-shaped channel, the second U-shaped channel configured to receive one or more additional teeth of the human, the second U-shaped channel being aligned with and opposing the first channel.

5. A device according to claim 4, wherein the first channel and second channel are at least partially defined by and separated by the bite plane (168).

6. A device according to claim 4, wherein all electrodes (124,128,132,136,140,144,148,152) disposed on the first surface of each channel are selectively activatable to the same polarity.

7. A device according to claim 6, wherein all electrodes (124, 128, 132, 136, 140, 144, 148, 152) disposed on the first surface of both channels are selectively activatable to the same polarity.

8. A device according to claim 4, where all electrodes (156) disposed on the second surface of each channel are selectively activatable to the same polarity.

9. A device according to claim 8, wherein all electrodes (156) disposed on the second surface of both channels are selectively activatable to the same polarity.

10. A device according to claim 1, wherein the electrically conductive polymer (120) comprises an electrically conductive silicone.

11. A device according to claim 1, wherein each electrode (124,128,132,136,140,144, 148,152,156) is selectively programmable to a high-impedance mode.

12. A device according to any preceding claim, wherein the plurality of electrodes (124, 128, 132, 136, 140, 144, 148, 152, 156) comprises a plurality of pairs of electrodes,
wherein each pair of electrodes is selectively activatable by the controller (300) separate and apart from any other electrode on the mouthpiece (100) .

13. A device according to claim 12, wherein the plurality of pairs is eight pairs.

14. A device according to claim 13, wherein four pairs are disposed along surfaces of each channel.

15. A device according to claim 1, wherein the first and second electrodes are selectively, simultaneously activatable in opposing polarities to establish a treatment zone (184, 186, 188, 190, 192, 194, 196, 198) .
